Europäisches Patentamt

European Patent Office

Office européen des brevets

(19)

(11) Publication number: **0 113 376**
A1

# EUROPEAN PATENT APPLICATION

published in accordance with Art. 158(3) EPC

(12)

(21) Application number: 83902123.5

(22) Date of filing: 30.06.83

Data of the international application taken as a basis:

(86) International application number:
PCT/JP83/00209

(87) International publication number:
WO84/00164 (19.01.84 84/02)

(51) Int. Cl.³: **C 07 C 85/26**
C 07 C 87/06, C 07 C 87/123

(30) Priority: 30.06.82 JP 114165/82

(43) Date of publication of application:
18.07.84 Bulletin 84/29

(84) Designated Contracting States:
FR GB

(71) Applicant: DAICEL CHEMICAL INDUSTRIES CO., LTD.
1, Teppo-cho
Sakai-shi Osaka 590(JP)

(72) Inventor: SHIMA, Koji 500-Banchi
Kamiyobe, Yobe-ku Himeji-shi
Hyogo-ken 671-12(JP)

(72) Inventor: NIWA, Hirotoshi
940-banchi Shinzaike Aboshi-ku
Himeji-shi Hyogo-ken 671-12(JP)

(74) Representative: Patentanwälte Grünecker, Dr.
Kinkeldey, Dr. Stockmair, Dr. Schumann, Jakob, Dr.
Bezold, Meister, Hilgers, Dr. Meyer-Plath
Maximilianstrasse 58
D-8000 München 22(DE)

(54) PROCESS FOR PURIFYING TERTIARY AMINE.

(57) In a process of washing an organic extract composed of a mixture of tertiary amine containing acids as impurities and an organic diluent with alkali to remove the impurities, an improvement is disclosed which comprises adjusting within a range of 3-6 the pH of the mixture being washed.

EP 0 113 376 A1

Croydon Printing Company Ltd

- DESCRIPTION

PROCESS FOR PURIFYING TERTIARY AMINE

Technical Field

The present invention relates to a process for purifying an organic extractant comprising a mixture of a tertiary amine and an organic diluent containing an acid as an impurity by washing the same with an alkali to remove the acid therefrom.

Background Art

A process for recovering an acid such as acetic acid, acrylic acid or nitric acid from an aqueous acid solution using an extractant comprising a tertiary amine and an organic diluent has been known. An aqueous acetic acid solution formed in industrial processes contains various impurities and when acetic acid migrates into a solvent phase in an extraction step, part of the impurities also migrates therein. After the extraction, the extract is washed with an alkali or distilled to separate the solvent from the acid and then the solvent is recycled. After the continuous use of the extractant by the recycling, the impurities in the aqueous acid solution and decomposition products from the extractant are accumulated gradually to cause a reduction in the extracting capacity. Therefore, these impurities must be removed from the recycle extractant.

An aqueous acetic acid solution discharged in a cellulose acetate production step contains sulfates derived from sulfuric acid used as a catalyst and impurities derived from pulp. Therefore, in a process for recovering acetic acid using an extractant containing the tertiary amine, the sulfates, the impurities due to pulp, derivatives of the tertiary amine and high-boiling products are accumulated in the recycle extractant over a long period of time to reduce the extracting capacity of the solvent. Accordingly, to maintain the impurity concentration of the recycle extractant below a given level, it has been considered to be a conventional process that part of the recycle solvent is taken out and subjected to a decontamination treatment. Various purification processes may be mentioned. However, in processes including a step of heating the solvent such as an evaporation or distillation step, high-boiling substances are formed in the purification step due to the decomposition of the tertiary amine, formation of amides and condensation of the diluent to make the distillation operation difficult and also the invite loss of the solvent.

Disclosure of Invention

After investigations made for the purpose of solving these problems, the inventors have found that the impurity formation in the heating step is caused by the presence of acids in the recycle solvent. The inventors have found also that the impurity formation can be reduced remarkably by washing the solvent with an aqueous alkali before the heating.

However, it has been found that in the neutralization and washing with the aqueous alkali solution, the mixture is emulsified by an alkali salt of a high-boiling acid or a quaternary salt of the amine in the washing step and, consequently, the oil/ water separation after the washing becomes quite difficult. The inventors tried to accelerate the separation by breaking the emulsion by various known processes such as a process wherein the separation of the two liquids are accelerated physically by centrifugation, a process wherein the emulsion phase is heated to accelerate the breakabe of emulsion drops and a process wherein the aggregation of emulsion drops is accelerated under a high voltage. However, the effects of these processes were yet unsatisfactory and economically satisfactory results could not be obtained.

Thus, it is quite difficult and economically disadvantageous to separate the formed emulsion into the respective phases. After intensive investigations of a process for washing the solvent with an alkali without forming the emulsion, the inventors have found a new purification process for a tertiary amine-containing organic extractant by removing an acid from the extractant. The present invention has been attained on the basis of this finding.

The present invention relates to a process for purifying a tertiary amine-containing organic extractant comprising a mixture of a tertiary amine and an organic diluent containing an acid as an impurity by washing the same with an alkali to remove the impurity, characterized in that the pH of the wash mixture is controlled to 3 to 6 in the washing step.

A process for recovering acetic acid from an acetic acid solution with an extractant comprising a tertiary amine and an organic diluent has been disclosed in, for example, the specification of the Japanese Patent Laid-Open No. 10131/1981.

The present invention will now be described in more detail by the best mode for carrying out the invention: acetic acid is extracted from the aqueous solution using, as an organic extractant, a mixture of a tertiary amine such as tri-n-octylamine (TOA) and an oxygen-containing organic solvent such as 3,3,5-trimethylcyclohexanone (TMCH) (both have boiling points higher than that of acetic acid) in an extractor devised to increase the dispersion/coalescence frequency of droplets, for example, a mixer-settler extractor. The obtained liquid extract is first freed of water which has been contained in the extract in a small amount in a dehydration/distillation column, and then introduced into an acetic acid recovery column, where acetic acid free of water is obtained from the top of the column and the regenerated extractant from the bottom. The separation of acetic acid from the extractant is smoothly effected by maintaining a temperature at the bottom of the column at around, for example, 140 to 150°C. Steam is utilized as the source of this heating as usual in chemical factories. Part of the regenerated extractant obtained from the bottom is introduced into the top of the dehydration/distillation column to be used as a reflux liquid therein. Although acetic acid is usually likely to go up in the column with water and the oxygen-containing compounds, it is extracted by the reflux liquid containing a tertiary amine and goes down, preventing the effusion of acetic acid out of the dehydration/distillation column. The remainder of the regenerated extractant (e.g. TOA and TMCH) obtained from the bottom of the acetic acid recovery column is recycled to the extractor of the first step.

The tertiary amines to be used are preferably those having a boiling point higher than that of acetic acid and form a non-aqueous phase. The number of carbon atoms contained in the tertiary amine should be around 12 to 40, considering the low solubility in the water phase and the separability from acetic acid by distillation. It is also preferable in order to obtain a large apparent partition coefficient that the tertiary amine has no large branches near the nitrogen atom. It is undesirable to have an ethyl or larger substituent group on a carbon atom neighboring the nitrogen atom by interposing one $CH_2$, not to mention adjacent to the nitrogen atom. It is also to be avoided to have a benzyl group or those having a cyclic structure near the nitrogen atom. In other words, the tertiary amines should be selected, from those which have a partial structure represented by $>N-CH_2-CR^1R^2$, wherein $R^1$ is a hydrogen atom and $R^2$ is a hydrogen atom or a methyl group, for example, $C_6$ or higher trialkylamines such as trihexylamine, trioctylamine, triisooctylamine (tris-2,4,4-trimethylpentylamine), trilaurylamine, dimethyllaurylamine, dimethylhexadecylamine, methyl-di(tridecyl)amine, or dimethyldecylamine; tertiary amines having an alkenyl group such as dimethyloleylamine or butylbis(5,5,7,7-tetramethyl-oct-2-en-1-yl)amine (XE-204); or tertiary amine mixtures such as dimethylcocoamine, dimethyl($C_{8-12}$ alkyl)-amines, or dimethyl(hydrogenated tallow)amine. Commercially available tertiary amines can be used as such. It is also possible to obtain tertiary amines by alkylating primary or secondary amines available as intermediates by a known method. Among many usable tertiary amines mentioned above, TOA is readily available and shows an excellent apparent partition coefficient when it is admixed with an oxygen-containing organic solvent to form an extractant.

Examples of the organic solvent to be used in combination with the amines are those oxygen-containing organic solvents which have a boiling point higher than acetic acid, e.g. ketones, alcohols, carboxylic esters, or phosphoric esters. When these solvents are used in combination with the tertiary amines described above, acetic acid in the aqueous solution can be extracted with a particularly large apparent partition coefficient.

The extractant containing the tertiary amine which is recycled in the recovery of acetic acid as described above contains an inorganic or organic acid as an impurity accumulated therein. The present invention provides a process for purifying the solvent by removing the impurity by washing with an alkali.

After investigations made for the purpose of preventing formation of the emulsion in the alkali washing step and loss of the tertiary amine and the organic diluent due to migration to the aqueous phase, the inventors have found that when the pH is controlled to 3 to 6 in the alkali washing, the inorganic and organic acids in the solvent are converted into their salts and the salts migrate selectively into the aqueous phase to permit the alkali washing without posing a problem of poor separability of the oil from the water and the loss of the tertiary amine due to migration thereof to the aqueous phase can be prevented completely. Accordingly, a time required for the separation of oil/water is reduced, a size of a settler in a mixer-settler extractor may be reduced and the loss of the tertiary amine and the organic diluent due to the emulsion formation in the aqueous phase can be eliminated. Therefore, the treatment by an activated sludge becomes unnecessary and problems such as environmental pollution can be exterminated.

Examples of the Invention

The following examples and comparative examples will further illustrate the present invention, which by no means limit the invention.

Example 1

A solvent mixture of tri-n-octylamine (TOA) and 3,5,5-trimethylcyclohexanone (TMCH) (volume ratio: 50/50) containing 1.5 wt.% of sulfuric acid used for the recovery of acetic acid by extraction at 40°C in a one-stage mixer-settler washing device was fed into a mixer at a rate of 400 g/h. A 5 wt.% aqueous sodium hydroxide solution was fed into the mixer from an aqueous alkali solution pump connected with a pH controller while the pH of the mixture in the mixer was controlled to 5 to 5.5.

The separability in the settler was high.
After the alkali washing, 406 g/h of the solvent
mixture containing 0.53 wt.% of water was recovered.
Sulfuric acid was not detected in the recovered,
high boiling extractant.  In the water phase discharged from the settler, 2000 ppm of the organic
diluent (TMCH) was contained in the form of a
solution but no tertiary amine (TOA) was detected.

Comparative Example 1

The same solvent mixture was fed into the
mixer at a rate of 400 g/h using the same device
as in Example 1.  The alkali washing was effected
while the pH was controlled to 7 to 8.

382 g/h of the solvent mixture containing 0.68
wt.% of water was recovered.  The dispersibility in
the settler was very poor.  The water phase discharged was milky and contained 1.8 wt.% each of
the tertiary amine and the organic diluent in the
form of an emulsion.

WHAT IS CLAIMED IS:

A process for purifying a tertiary amine-
containing organic extractant comprising a mixture
of a tertiary amine and an organic diluent containing an acid as an impurity by washing the same with
an alkali to remove the impurity, characterized in
that the pH of the wash mixture is controlled to 3
to 6 in the washing step.

# INTERNATIONAL SEARCH REPORT

**0113376**

International Application No. PCT/JP83/00209

## I. CLASSIFICATION OF SUBJECT MATTER (if several classification symbols apply, indicate all) [3]

According to International Patent Classification (IPC) or to both National Classification and IPC

Int. Cl.[3]   C07C 85/26, 87/06, 87/123

## II. FIELDS SEARCHED

Minimum Documentation Searched [4]

| Classification System | Classification Symbols |
|---|---|
| I P C | C07C 85/26, 87/06, 87/123 |

Documentation Searched other than Minimum Documentation
to the Extent that such Documents are Included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| | | |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [3] |
|---|---|
| September 26, 1983  (26. 09. 83) | September 26, 1983  (26. 09. 83) |

| International Searching Authority [1] | Signature of Authorized Officer [20] |
|---|---|
| Japanese Patent Office | |

Form PCT-ISA/210 (second sheet) (October 1981)